Europäisches Patentamt

European Patent Office

Office européen des brevets

(11) Publication number: **0 431 189 A1**

## (12) EUROPEAN PATENT APPLICATION
### published in accordance with Art. 158(3) EPC

(21) Application number: 90909387.4

(22) Date of filing: 25.06.90

(86) International application number:
PCT/JP90/00822

(87) International publication number:
WO 91/00517 (10.01.91 91/02)

(51) Int. Cl.⁵: **G01N 33/00**

(30) Priority: 30.06.89 JP 166646/89

(43) Date of publication of application:
**12.06.91 Bulletin 91/24**

(84) Designated Contracting States:
**DE FR GB**

(71) Applicant: **HITACHI, LTD.**
**6, Kanda Surugadai 4-chome**
**Chiyoda-ku, Tokyo 100(JP)**

(72) Inventor: **IRIE, Ryotaro**
**3-5-14-305, Shirahata Urawa-shi**
**Saitama-ken 336(JP)**

(74) Representative: **Strehl, Schübel-Hopf,**
**Groening**
**Maximilianstrasse 54 Postfach 22 14 55**
**W-8000 München 22(DE)**

(54) **METHOD OF ASSISTING MATERIAL DESIGN AND APPARATUS THEREFOR.**

(57) A method of assisting material design and an apparatus therefor wherein one-body potential of a carrier in a neutral isolated state such as an electron, of an assumed system, namely, common one-body potential (independent of arrangement information of the above carriers) with respect to the above carriers is determined using the Schrödinger equation, the row element of one-electron Hamiltonian is derived therefrom, and the energy of the system is efficiently found by the approximation of many-body perturbation theory, etc.

FIG. 1

START → 101 READING AN ATOMIC CONFIGURATION → 102 GENERATION OF MATRIX ELEMENTS OF LEAF ONE-ELECTRON HAMILTONIAN → 103 CALCULATION AND OUTPUTTING THE ELECTRON ORBITAL FUNCTIONS → 104 READING ELECTRONIC CONFIGURATION → 105 CALCULATION OF THE RESPECTIVE EIGEN VALUES OF THE UNPERTURBED TERM (E₀) FOR ELECTRONIC CONFIGURATIONS → 106 CALCULATION OF THE RESPECTIVE EXPECTATION VALUE OF THE PERTURBATION TERM (E₁) FOR ELECTRONIC CONFIGURATIONS → 107 TOTAL-ENERGY CALCULATION BY ADDING E₁ TO E₀ → 108 OUTPUTTING THE RESPECTIVE TOTAL ENERGIES FOR ELECTRONIC CONFIGURATIONS → END

EP 0 431 189 A1

## A MATERIAL-DESIGN-SUPPORTING METHOD AND APPARATUS

Technological Field

The present invention relates to a material design supporting method and apparatus, and more particularly to a material design supporting method and apparatus suited for supporting a material user in the design of materials for a variety of purposes such as selection of various kinds of materials, particularly medicines, organic materials, and electronic materials.

Background of the Invention

In analyzing the electronic structure of molecules and clusters and the physical properties of molecules closely associated with electron wave, independent particle models are essential. The extended Hueckel method (R. Hoffmann, The Journal of Chemical Physics, Vol.39, No. 6, 1963, pages 1397-14l2), an application of the independent particle model to a molecular electronic structure calculation method, requires the least calculating effort and has the simplest theoretical structure among the available methods for calculating and analyzing the material's electronic structure (molecular orbital method). Thus, the extended Hueckel method has been considered the most suited for, and actually applied to the search and design of materials.

With the traditional extended Hueckel method, the electronic structure is calculated by using empirically obtained matrix elements for one-electron Hamiltonian (Coulomb integral and resonance integral) without explicitly assuming the electron potential. Recently, however, the inventors of this invention proposed a one-electron Hamiltonian based on the Wigner-Seitz-type potential (LEAF) and successfully derived its matrix elements. The inventors also derived the matrix elements for the extended Hueckel method with approximation added. This means that the extended Hueckel method is one of the approximated solutions to the molecular orbital method based on the LEAF potential. The LEAF molecular orbital method is found to have a characteristic that the energy of the bound-state electronic orbital, when calculated by this method, always results in a negative value. This is a physically normal characteristic, not found with the extended Hueckel method. (R. Irie, Theoretica Chimica Acta, Vol.70, pages, 1986, 239-252).

In the traditional extended Hueckel method, the complete Hamiltonian H, a total-energy operator of a molecule (or a cluster), is given as a sum of one-electron Hamiltonians h(i) for all electrons in the system.

$$H = \sum_i h(i) \qquad \cdots\cdots\cdots\cdots\cdots\cdots\cdots (1)$$

This is one of the factors that simplify the theoretical structure. This equation has no quantitative ground and does not necessarily reflect an observed fact. In other words, since the conventional extended Hueckel method cannot take into account the difference $\Delta$ between the correct complete Hamiltonian H and the summation $\Sigma h(i)$, it lacks a quantitative aspect and does not necessarily reflect the observed fact.

As a method to solve this problem by improving the quantitative nature, the Hartree-Fock method and the CI method (A. Szabo, N. S. Ostlund, Modern Quantum Chemistry, Macmillan, 1982, pages 53-55 and 60-64) are known. However, since the Hartree-Fock method optimizes the one-electron Hamiltonian h(i) for one particular electronic configuration, the absolute value of the expectation value for the difference $\Delta$, though it may not be large for that electronic configuration, will become very large for other electronic configurations because h(i) would sometimes overestimate the number of electrons in the system.

$$\Delta = H - \sum_i h(i) \qquad \cdots\cdots\cdots\cdots\cdots\cdots (2)$$

Because it is practically impossible to determine a physical quantity from an unrealistic zero-order approximation model in which the electron number is overestimated, the difference $\Delta$ cannot be handled as the perturbation term for all electronic states. That is, the electronic structure analysis is complex and the calculation takes long so that the Hartree-Fock method is not suited for material design nor material searching.

On the other hand, the CI method, which improves the accuracy of total-energy calculated by the Hartree-Fock method, needs the laborious calculation and is time-consuming much more than the Hartree-Fock method. Thus the CI method is not suited for material design nor material searching either.

Disclosure of the Invention

The object of the present invention is to provide a material design supporting method and apparatus which solves the problems experienced with the prior art and which has improved usefulness and reliability. More specifically, it is the object of the invention to provide a material design supporting method and apparatus which has a sufficiently high precision for material design and material searching and has a practical processing speed for material searching.

Another object of the invention is to propose a total energy calculation method - which has a simple theoretical structure (equation (1)) applicable to material design - by introducing a perturbation term definable for any electronic configuration which is defined by an appropriately proposed one-electron Hamiltonian. And a further object is to provide a material design supporting method and apparatus which employs the total-energy calculation method proposed.

To achieve these and other objectives, the present invention provides a material design supporting method and apparatus which introduces h(i) that enables the approximated relationship of equation (1) to hold not only for a particular electronic configuration but for every configuration; and which employs a perturbation term definable for all electronic configurations that exist in the system and thereby performs the numerical analysis by approximating the perturbation term to calculate the complete Hamiltonian of the system.

That is, a first aspect of the invention provides a material design supporting method and apparatus which has a sufficient precision for material design and searching and has a practical processing speed for material searching, and which consists of the steps of:

inputting a first parameter group that define atomic configuration of a system made up of a plurality of atoms;

inputting a second parameter group that defines the configurations of carriers such as electrons in the system;

determining, by using the first parameter group, matrix elements of one-particle Hamiltonian that are generated from one-particle potential of carriers, common to said carrier configurations;

determining orbitals of the carriers by solving the eigen value problem of the one-particle Hamiltonian;

calculating, for the carrier configurations, eigen values of unperturbed term that constitutes the complete Hamiltonian of the system, by using the carrier orbitals and the second parameter group;

calculating, for the carrier configurations, expectation values of the perturbation term that constitutes the complete Hamiltonian of the system, by using the carrier orbitals and the second parameter group; and

displaying the calculated results of the eigen values and the expectation values.

The system consisting of a plurality of atoms may include, for example, molecules such as organic compounds or inorganic compounds, or clusters. This invention can also deal with high polymer compounds such as protein in particular. The first parameter group that defines the atomic configuration of such a system includes the number of atoms making up the system to be analyzed and atomic numbers and positions of these atoms. Where internal parameters are not used in the step of determining the one-particle Hamiltonian as described later, the first parameter group includes the number of particles in the valence shell, the number of valence electrons, the number of electrons in core shells, principal quantum numbers of atomic orbitals forming each atomic shell, azimuthal quantum numbers, atomic orbital energies, the number of Slater-type orbitals (STOs) making up the atomic orbitals, STO exponents, STO coefficients, and intra-atomic electron-electron repulsion energy.

The second parameter group that defines a plurality of configurations of carriers such as electrons in the system includes, for instance, the number of electrons occupying each orbital.

The one-particle potential common to the carrier configurations, determined from the first parameter group, introduces a potential similar to the one-particle potential in the Wigner-Seitz cell method in the solid state physics theory. (In this specification, this is called a "Linkage of Embedded Atomic Fields, or LEAF, potential" and a one-particle Hamiltonian generated by using this potential is referred to as an "LEAF Hamiltonian.") This potential is defined as one-particle potentials of free atoms linked together. (In this sense this potential may be called a Wigner-Seitz-type potential.) As detailed later, however, this invention is characterized by the use of one-particle potential that does not depend on the carrier configurations. The second feature is that a cell containing each atom which is employed in this invention in connection with the one-particle potential is different from the cell used in the Wigner-Seitz cell method and is not defined by three-dimensional geometry. For example, the cell is defined by the following three conditions:

3

1. A single cell includes the three-dimensional region where the carrier density of free and neutral atom contained in the cell is high;
2. Cells do not overlap each other; and
3. All the cells in the system combined fill the entire three-dimensional space where the carriers can exist.

The one-particle potential common to carrier configurations, which is determined as mentioned above, is used to form the one-particle Hamiltonian for the carriers. In other words, the one-particle Hamiltonian operator is determined by adding the kinetic energy of a carrier to the one-particle potential obtained above. The eigen value problem for the Hamiltonian can ordinarily be solved as a simple matrix diagonalization calculation problem by performing approximation, which is carried out by expanding the one-particle Hamiltonian with the Hartree-Fock atomic orbitals of neutral and free atoms in the system to determine the carrier orbitals. In using the solution of this example, if there is no numerical error during the process of calculation, the orbital energies are always calculated as values less than 0 eV. Even when the numerical errors occurring during the computation cannot be neglected, the orbital energies are calculated to be less than +0.1 eV.

One of the features of particular importance in this invention is that the carrier orbitals are determined without using the information on the carrier configurations, i.e. without the above-mentioned second parameter groups. Therefore, the following steps are taken. The information on the atomic configurations in the system which have been input in advance - the carrier orbitals determined by the first parameter group - are stored in memory. And by inputting various kinds of carrier configuration information (second parameter groups), it is possible to determine very easily the system energy for each of the individual carrier configurations by using the carrier orbitals stored in memory, thus substantially improving the time efficiency.

As is seen from the above explanation, it is not necessary with this invention to enter the second parameter groups into the computer from the beginning. The only requirement is that the information be able to be used before calculating the system energy.

In determining the total energy of the system by using the carrier orbitals and the second parameter groups, this invention calculates the eigen value problem of the complete Hamiltonian operator of the system by using the perturbation approximation. The complete Hamiltonian operator is given as the sum of the kinetic energy operators for all carriers in the system and the potentials of these carriers. Those potentials may preferably be taken as the sum of potential energies derived from the Coulomb interactions between the atomic nuclei fixed in space and the carriers, between the carriers themselves, and between the atomic nuclei themselves. The effective perturbation approximation involves selecting from the complete Hamiltonian operator a term made up of one-particle Hamiltonians of the above-mentioned carriers as an unperturbed term and determining the eigen values for the plurality of carrier configurations.

By using the aforementioned carrier orbitals and the second parameter groups, the term that remains after subtracting the unperturbed term from the complete Hamiltonian of the system is taken as a perturbation term, and the correction value for this term is calculated for the plurality of carrier configurations (many-body perturbation approximation). It is thus possible to introduce appropriate approximation (described later) into the perturbation term. Particularly the first-order perturbation approximation is effective.

The eigen values and correction values thus determined are output on the output display device as individual values or the sum of these values.

Another aspect of this invention provides a material design supporting method which has a sufficiently high precision for material design and searching and has a calculation speed applicable to the material searching, and which comprises:

a first step of determining carrier orbitals in the system in advance and independently of the information on the configurations of the carriers existing in the system, by using a first parameter group defining the atomic configuration in the system made up of a plurality of atoms; and

a second step of determining the energy of the system from a second parameter group defining a plurality of configurations of carriers such as electrons in the system and also from said carrier orbitals obtained in the first step.

According to a limited aspect of this invention, the second step provides a material design supporting method to determine the energy of the system by using the perturbation approximation.

According to another limited aspect of this invention, the above second step provides a material design supporting method which has a step of selecting as an unperturbed term for the perturbation approximation the term in the complete Hamiltonian operator of the system that is represented by the sum of the one-particle Hamiltonians. With the unperturbed term thus selected for the perturbation approximation, it is possible to reduce the calculation time drastically while maintaining the precision required for the material design and searching at a certain level.

Still another aspect of this invention provides a material design supporting apparatus which has a sufficient precision for material design and searching and a practical calculation speed for material searching, and which comprises:

a means for inputting a first parameter group defining an atomic configuration of a system made up of a plurality of atoms;

a means for inputting a second parameter group defining configurations of a plurality of carriers such as electrons in the system;

a total energy calculation processing section to calculate the total energy of the system, said total energy calculation processing section further comprising:

a means for determining a one-particle potential of the carriers which is common to a plurality of carrier configurations, by using the first parameter group;

a means for determining orbitals of the carriers by using the one-particle potential;

a means for calculating eigen values of an unperturbed term making up the complete Hamiltonian of the system for the plurality of carrier configurations by using the carrier orbitals and the second parameter group; and

a means for calculating expectation values of a perturbation term making up the complete Hamiltonian of the system for the plurality of carrier configurations by using the carrier orbitals and the second parameter group; and

a means for displaying the calculated results of the eigen values and the expectation values.

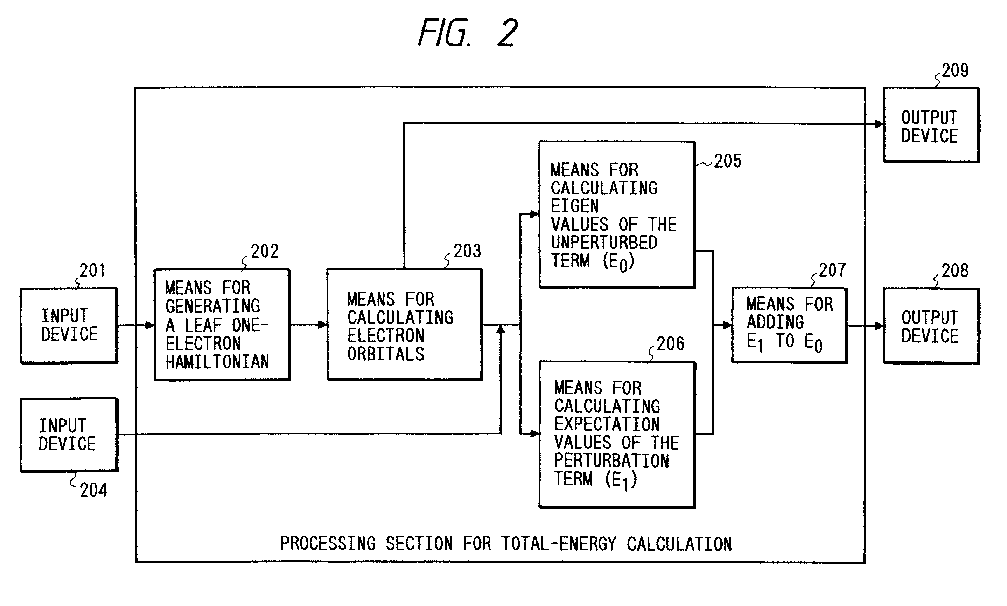

Figure 2 shows the total energy calculation processing section and the input and output means in the material design supporting apparatus according to this invention. In this invention, a system for which the energy calculation can be carried out (typically a molecular structure) is not limited to those having electrons as carriers. However, since in the systems that require the energy calculation the carriers are generally electrons, the following explanation takes electrons as carriers.

In Figure 2, reference numeral 201 represents an input device such as a keyboard to enter input data (first parameter group) including coordinates of nuclei in a system fixed in space (atomic configuration). Denoted 202 is a means to determine matrix elements of one-particle Hamiltonian - LEAF one-electron Hamiltonian - for the carriers (electrons) by using the aforementioned Wigner-Seitz-type potential. In this invention, the LEAF one-electron Hamiltonian can be obtained from the first parameter group. In other words, it can be determined independently of the information (second parameter group) on the electronic configurations present in the system that are used for the energy calculation. Designated 203 is a means to calculate electron orbitals in the system by diagonalizing the matrix derived from the one-particle Hamiltonian matrix. In the invention, up to this point, the processing proceeds without using any particular information on the electronic configurations in the system. Thus, where a variety of electronic configurations are considered for a system with the same atomic configuration commonly used, this invention permits a particularly efficient energy calculation. That is, once the electron orbitals have been determined from data on the atomic configuration of the subject system, it is stored in memory so that it can be read out at any of the succeeding steps.

The electron orbitals thus obtained and the input data containing electronic configurations supplied as the second parameter group from the input means 201 or separate input means 204 are used to calculate the energy of the system by perturbation approximation. The material design supporting apparatus of this invention includes a means 205 and a means 206. The means 205 calculates eigen values for unperturbed term in the complete Hamiltonian of the system, for example, eigen values for the sum of the LEAF one-electron Hamiltonians for all the electrons in the system. The means 206 calculates expectation values for the term in the complete Hamiltonian that remains when the unperturbed term - for example, the sum of the LEAF one-electron Hamiltonians for all the electrons in the system - is subtracted from the complete Hamiltonian. The obtained eigen values for the unperturbed term and the expectation values for the perturbation term are added together by an adding means 207 and output to an appropriate output display means 208, such as display, as an output data containing the total energy of the system. It may be appropriate, if necessary, to output to the output device 208 or separate output device 209 output data containing electron orbitals information along with output data containing the total energy of the system. The outputting of the electron orbitals information is useful in the material design.

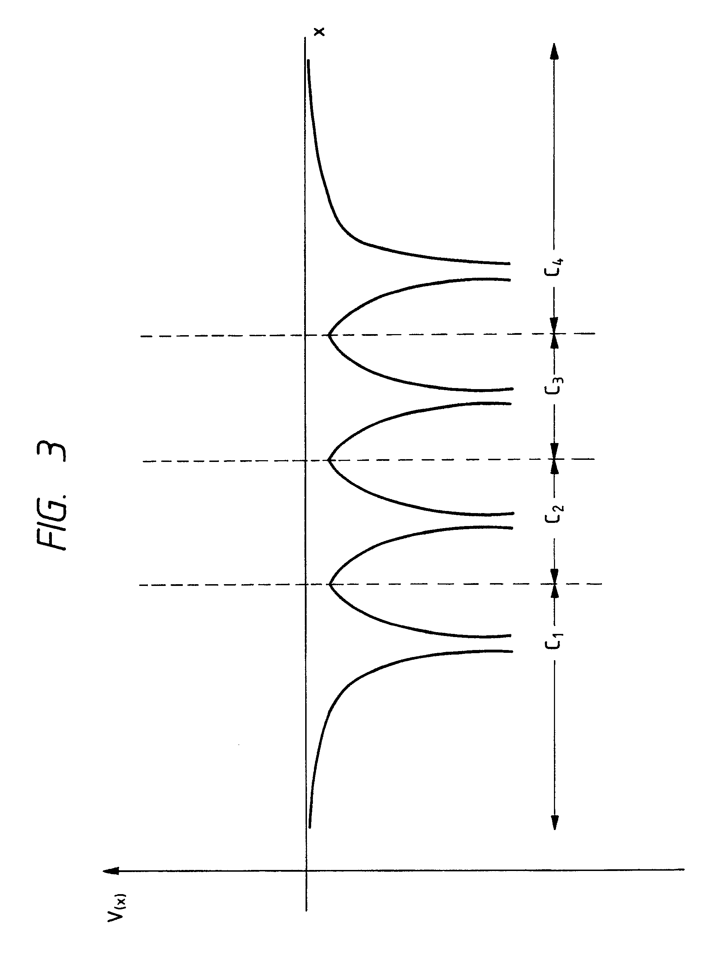

Figure 3 illustrates the one-electron potential used in this invention for the case of an aggregate of hydrogen atoms ($H_4$). In Figure 3, the abscissa $x$ represents the position coordinates of electrons; the ordinate $V(x)$ one-electron potential energy; and $C_1$ $C_2$, $C_3$ and $C_4$ atomic cells of the hydrogen atoms 1, 2, 3 and 4. Curves in individual atomic cells indicate the potentials $V(x)$ of electrons of free hydrogen atoms.

The linkage of one-particle potential functions of the free atoms defined in individual atomic cells, as shown in Figure 3, or its approximation, is called an LEAF potential. The one-particle potentials of the free atoms comprise the repulsion potential from other electrons of the neutral and free atoms and attraction from the nuclei. However, the repulsion potential from other electrons is peculiar to each atom and thus defined independently of the input data containing the electronic configurations. Therefore in this invention, as mentioned earlier, it is possible to enter this information as the first parameter group defining the atomic configuration.

The LEAF potential added with the kinetic energy operator of the electrons is the LEAF Hamiltonian.

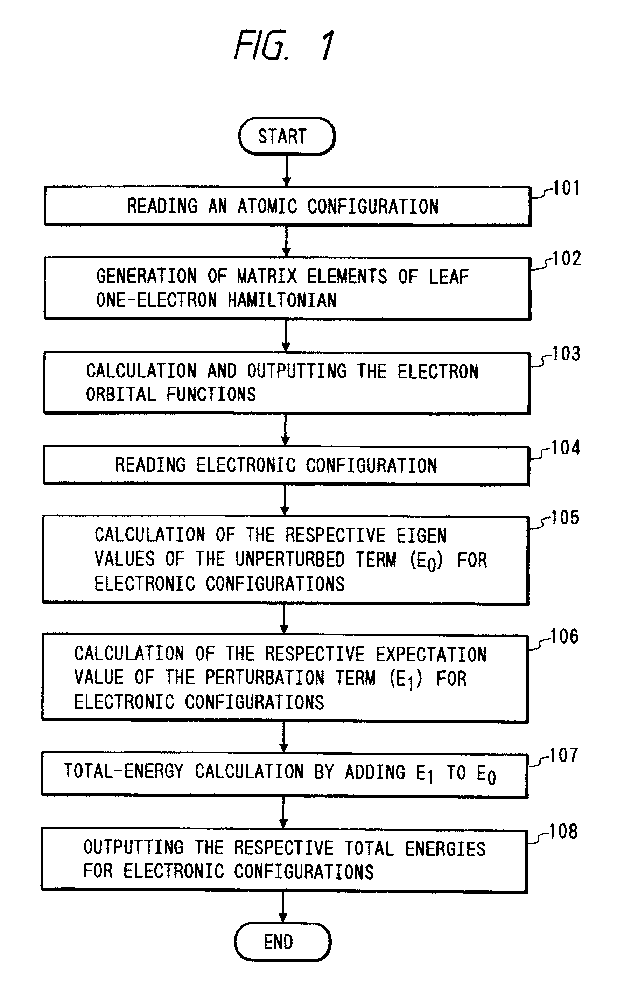

Figure 1 is a flowchart showing the sequence of operation performed in the material design supporting method of this invention. The first parameter group or data on the atomic configuration of a system is read in from the input means 201 of Figure 2 into the means 202 which generates the one-electron Hamiltonian of Figure 2 (processing 101). Using the atomic configuration data read in by the LEAF one-electron Hamiltonian generating means 202 and the parameters (principal quantum number, azimuthal quantum number, or Slater-type orbital exponent and coefficient) representing the atomic orbitals incorporated (or read in by the input means 201), the overlap integral and the intra-cell integral are calculated. These integrated values and the incorporated atomic orbital energy (or read in from the input means 201) are subjected to such processing as multiplication, addition and subtraction to determine the one-particle Hamiltonian matrix:

$$h = SWeS$$

where S is a matrix of the overlap integral, W is a diagonal matrix of the intra-cell integral, and e is a diagonal matrix of the atomic orbital energy (processing 102).

Then, the electron orbitals calculating means 203 of Figure 2 determines the electron orbitals and their energies by using the square root of the matrix of overlap integral and transforming the matrix (h) generated by the processing 102 into the diagonal matrix. Then the calculated electron orbitals and their energies are output to the following three means: to the means 205 of Figure 2, which calculates eigen values for the unperturbed term in the total Hamiltonian of the system; to the means 206, which calculates expectation values for the perturbation term that results from subtracting the unperturbed term from the complete Hamiltonian; and also to the output display means 209 (processing 103). The electronic configuration information is read in from the input means 204 of Figure 2 into the means 205 which calculates eigen values for the unperturbed term in the complete Hamiltonian of the system and into the means 206 which calculates expectation values for the perturbation term in the total Hamiltonian (processing 104). Then the means 205 for calculating eigen values for unperturbed term in the total Hamiltonian of the system processes the electronic configuration information read in ($v_1$, $v_2$, ..., $v_d$) and the electron orbitals energies ($\epsilon_1$, $\epsilon_2$, $\epsilon_d$) determined by the electron orbital calculating means 203 to calculate the eigen values (processing 105)

$$E_0 = \sum_{i=1}^{d} v_i \, \epsilon_i$$

The means 206 of Figure 2 for calculating the perturbation term that results from subtracting the unperturbed term from the complete Hamiltonian processes the electronic configuration information read in and the electron orbitals energies to calculate the expectation values for the perturbation term H' (processing 106)

$$E_1 = \int \Phi^* H' \Phi \, d\tau$$

where $\Phi$ is a state function of electronic configuration and $\tau$ is the coordinates of space, and the integration is performed over the space. The processing 107 calculates the total energy by adding $E_0$ and $E_1$ by the adding means 207 of Figure 2.

The total energy obtained by the adding means 207 is output to the output display means 208 of Figure 2 (processing 108).

To achieve the above objective, the total energy calculation processing section employs, as shown in Figure 2, the LEAF Hamiltonian generating means 202 to generate one-electron Hamiltonian, and incorporates the means 206 for calculating the expectation values of perturbation term and the means 207 for adding the expectation values of perturbation term to the eigen values of unperturbed term.

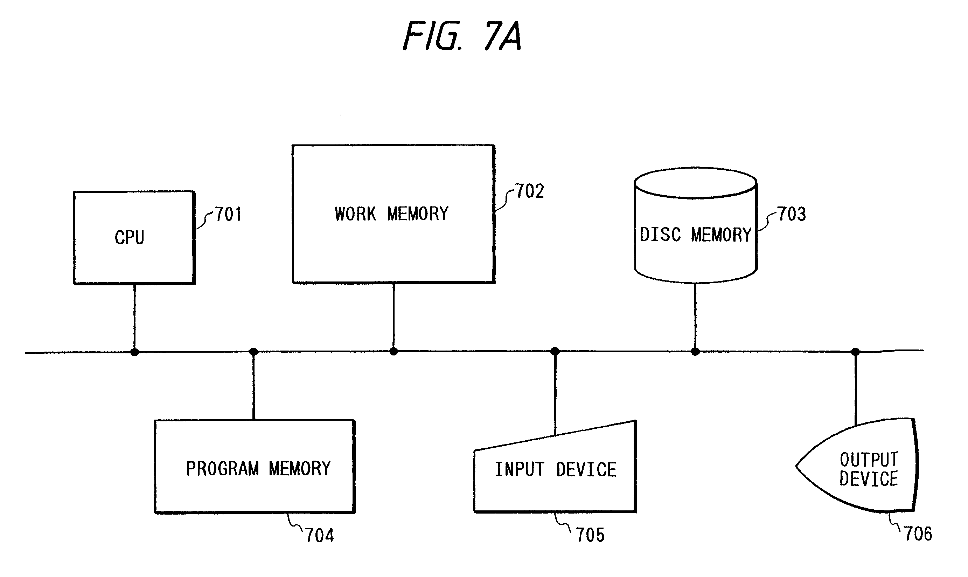

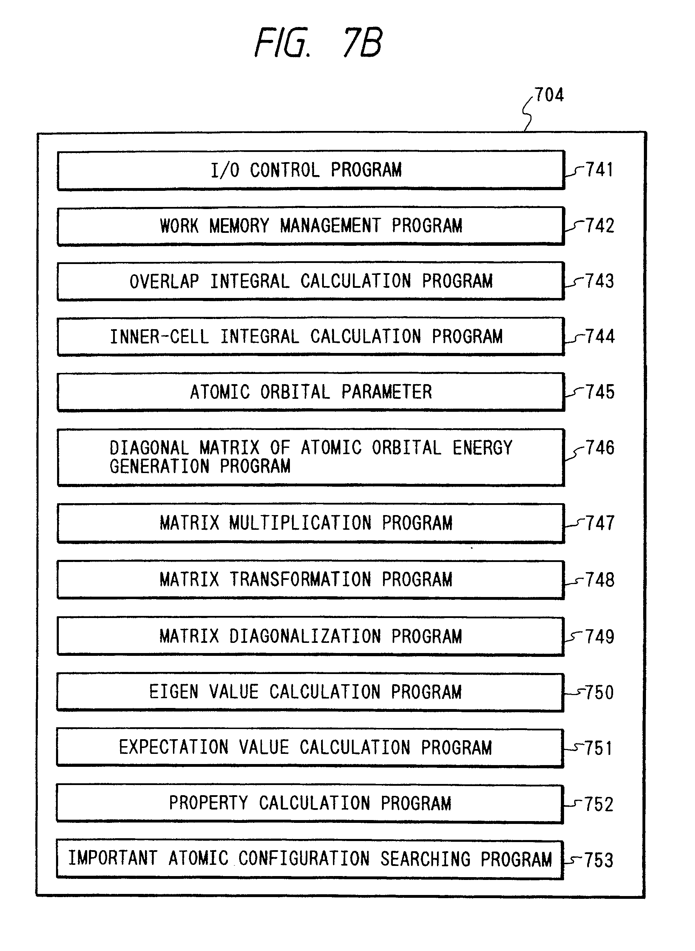

Next, the flow of the material design supporting method of this invention will be explained by referring to the apparatus configuration in Figure 7A and the program configuration in Figure 7B of the invention.

The atomic configuration data or the first parameter group is read from input device 705 such as keyboard or from auxiliary memory 703 such as disc into work memory 702 as shown in Figure 7A (processing 101). Input and output of the input data and output data used in this invention is controlled by an I/O control program 741 to control the keyboard 705 as the input device and a display 706 as the output device. When any change is to be made of atomic orbital parameters 745 incorporated in the program memory 704 or of the atomic orbital energy, the changed portion is entered into the work memory 702 from the keyboard 705 or disc 703. The work memory 702 is controlled by a work memory management program 742 stored in the program memory 704. While in the drawing the program memory 704 and the work memory 702 are shown as separate devices, these memory are generally provided as different memory areas in one memory.

By using the atomic configuration data stored in the work memory 702, changed atomic orbital parameters, overlap integral calculation program 743 inner-cell integral calculation program 744, both programs contained in the program memory 704, and the atomic orbital parameter 745 incorporated in the program memory 704, the CPU 701 calculates the matrix of overlap integral and the diagonal matrix of intra-cell integral and stores the results in the work memory 702. The CPU 701 then generates a diagonal matrix of atomic orbital energy by using the modified atomic orbital energy, the atomic configuration data, the atomic orbital energy in the atomic orbital parameter 745 incorporated in the program memory 704 and the atomic orbital energy diagonal matrix generating program 746 in the program memory 704. The diagonal matrix thus obtained is stored in the work memory 702.

By using the matrix multiplication program 747 stored in the program memory 704, the CPU 701 also computes the matrix of one-particle Hamiltonian

$$h = SWeS$$

where S is the overlap integral matrix, W the intra-cell integral diagonal matrix and e the diagonal matrix of atomic orbital energy. The resultant is stored in the work memory 702 (processing 102).

The next step is for the CPU 701 to determine the electron orbitals and electron orbital energies by using the one-particle Hamiltonian matrix and the overlap integral matrix stored in the work memory 702, and a matrix transformation program 748 that takes square root of the overlap integral and a matrix diagonalization program 749, both stored in the program memory 704. The CPU 701 then stores the calculated results in the work memory and outputs them to the display 706 and disc 703 as required (processing 103).

Next, the electronic configuration information is taken into the work memory 702 from the keyboard 705 or disc 703 (processing 104).

Then, according to an unperturbed term eigen value calculating program 750 stored in the program memory 704, the CPU 701 processes the electronic configuration information $v_1$, $v_2$, ..., $v_d$ and the electronic orbital energies $\epsilon_1$, $\epsilon_2$, ...,$\epsilon_d$ stored in the work memory 702 to calculate the eigen values of unperturbed term

$$E_0 = \sum_{i=1}^{d} v_i \epsilon_i$$

and then stores the calculated result in the work memory 702 (processing 105).

Using the electronic configuration information, electron orbitals and atomic configuration data, all stored in the work memory 702, the CPU 701 calculates the expectation values $E_1$ for the perturbation term - the term that results from subtracting the unperturbed term from the complete Hamiltonian - and stores them in the work memory 702 according to a perturbation term expectation value calculating program 751 stored in the program memory 704 (processing 106).

In the processing 107, the CPU 701 uses the addition program 752 stored in the program memory 704 to calculate the total energy by adding $E_1$, to $E_0$ and then stores the calculated total energy in the work memory 702.

The total energy stored in the work memory is either stored in the disc 703 or output to the display 706 (processing 108).

Figure 7B shows a property calculation program 752 and an important atomic configuration searching program 753. These will be detailed in the following section titled the "best mode of embodiment for implementing the invention." One of the features of this invention, as mentioned above, includes the steps for the calculation of the total energy of a system and the apparatus for carrying out these steps. Some examples that make effective use of the feature are illustrated in the following embodiments.

According to a further aspect of this invention, the processing 106 of Figure 1 approximates the perturbation term with the function of spin number in each atom for the purpose of reducing the amount of calculation work and simplifying the theoretical expression. In determining the expectation value (spin density) of the spin number in each atom, the Mulliken population analysis may be used.

According to a further aspect of this invention, in applications where the spin is not important, further simplification of the theoretical expression can be obtained by approximating the perturbation term with the number of electrons in each atom during the processing 106 of Figure 1. The Mulliken population analysis may be employed in determining the expectation value for the electron number in each atom.

With this invention, it is possible to realize a reliable material design support which maintains clarity and simplicity desired for the material design while requiring less of the calculating work.

Furthermore, this invention enables efficient energy calculation for a variety of electronic configurations in a system.

Other features and advantages of the invention will become apparent to a person skilled in the art when he or she reads a section titled "best mode of embodiment for implementing the invention," which is given later on.

Brief Description of the Drawings

Figure 1 is a flowchart showing the sequence of processing as performed by an energy calculation processing section according to this invention;

Figure 2 is a block diagram showing a total energy calculation processing section according to the invention;

Figure 3 is a graph schematically illustrating one example of LEAF potential adopted by the total energy calculation processing section according to the invention;

Figure 4 is a block diagram showing the overall configuration of one embodiment of a material design supporting apparatus according to the invention;

Figure 5 is a graph showing a calculation result of the total energy of hydrogen fluoride obtained according to the invention;

Figure 6 is a graph showing a calculation result of the total energy of arsine obtained according to the invention;

Figure 7A is a block diagram of one example configuration of a calculating apparatus shown for the purpose of explaining the flow of operation performed by the material design supporting method of this invention; and

Figure 7B is a diagram showing one example configuration of a program memory employed in the material design supporting method and apparatus of the invention.

Best Mode of Embodiment for Implementing the Invention

This invention incorporates the expectation calculation means 206 and the addition means 207 of Figure 2, both of which take as a perturbation term the difference $\Delta$ between the correct complete Hamiltonian H and the sum of the LEAF one-electron Hamiltonians

$$\sum_i (i)$$

and introduce a correction term according to the first-order perturbation theory. Thus, compared with the conventional extended Hueckel method that does not include the expectation value calculation means 206 and the addition means 207, the method of this invention has an improved quantitative characteristic. When compared with the Hartree-Fock method, since this invention employs the LEAF Hamiltonian (means 202 of Figure 2 for calculating the matrix elements of one-particle Hamiltonian) as a one-electron Hamiltonian generating means, which is independent of the electronic configurations and therefore does not overvalue nor undervalue other-electron numbers, the first order correction provided by the expectation value calculation means 206 and the addition means 207 of Figure 2 is applicable for all electronic configurations. Moreover, the first-order correction given by these means 206, 207 can be obtained simply by calculating the expectation values of the perturbation term $\Delta$

$$\int \Phi^* \Delta \Phi \, d\tau$$

and by adding the expectation values to the eigen values of unperturbed term. Hence the amount of calculation work is not large, as in the case of the extended Hueckel method. Since the expectation values can be approximated as a quadratic function of charges of atom, and since the relationship between the total Hamiltonian H and the one-electron Hamiltonian h(i) is clearly defined, the analysis of the total energy can easily be performed by the user after the output result is obtained by this method.

As is seen from the foregoing, the material design supporting apparatus of this invention including the total energy calculation processing section has a sufficient level of accuracy for material design and searching, a calculation speed applicable to the material searching, and also a simple theoretical formulation (equation (1)) applicable to the material design. This method is superior to the extended Hueckel method in terms of precision and to the Hartree-Fock method and CI method in terms of the calculation speed and the simplicity of theoretical structure.

In applications where the spin is not important, the theoretical equation can be further simplified. For that purpose, the perturbation term $\Delta$ can be approximated by the expectation value calculating means 206 of Figure 2 with a function of electronic number on each atom ($K(\alpha)$), as follows:

$$
\begin{aligned}
H = {} & \sum_i h(i) \\
& + \sum_\alpha (1/2)K(\alpha)(K(\alpha)+1)\gamma(\alpha) \\
& + \sum_{\alpha<\beta} K(\alpha)K(\beta)/f(R_{\alpha\beta}) \\
& + (\text{constant}) \quad\quad\quad \ldots\ldots\ldots\ldots\ldots\ldots (3)
\end{aligned}
$$

where $\gamma$ is an intra-atomic electron-electron repulsion energy, $f(R_{\alpha\beta})$ is a function of the distance $R_{\alpha\beta}$ between an $\alpha$-th atom and a $\beta$-th atom. For instance,

$$
f(R_{\alpha\beta}) = \left\{
\begin{array}{ll}
R_{\alpha\beta} & (R_{\alpha\beta} > R_{\alpha\beta}{}^\circ) \\[2mm]
R_{\alpha\beta}{}^\circ & (R_{\alpha\beta} \leq R_{\alpha\beta}{}^\circ)
\end{array}
\right. \quad\quad \ldots\ldots\ldots\ldots (4)
$$

where $R_{\alpha\beta}{}^\circ$ is the shortest distance between the $\alpha$-th and $\beta$-th atoms. Therefore, according to the first-order perturbation theory, the total energy is given by

$$
\begin{aligned}
E = {} & \sum_i \in (i) \\
& + \sum_\alpha (1/2)Q(\alpha)(Q(\alpha)-1)\gamma(\alpha) \\
& + \sum_{\alpha<\beta} Q(\alpha)Q(\beta)/f(R_{\alpha\beta}) \\
& + (\text{constant}) \quad\quad\quad \ldots\ldots\ldots\ldots\ldots\ldots (5)
\end{aligned}
$$

where $\epsilon(i)$ is an occupied electron orbital energy and $Q(\alpha)$ is a charge of each atom. As can be seen from equation (5), since the first-order correction term that considers the charge distribution in the system is given by the expectation value calculation means 206 and the addition means 207, the quantitative characteristic is improved. Further, the first-order correction term is a simple function of the charge of each atom, a quantity that can be grasped intuitively, making it very simple for the user to analyze the relationship between E and $\Sigma\epsilon$, that is, between H and $\Sigma$h after being output on the output device. Hence, the material design supporting method and apparatus of this invention which includes the total energy calculation processing section is all the more suited for the material design and material searching.

Now, some example embodiments of this invention will be described by referring to the attached drawings.

[First Embodiment]

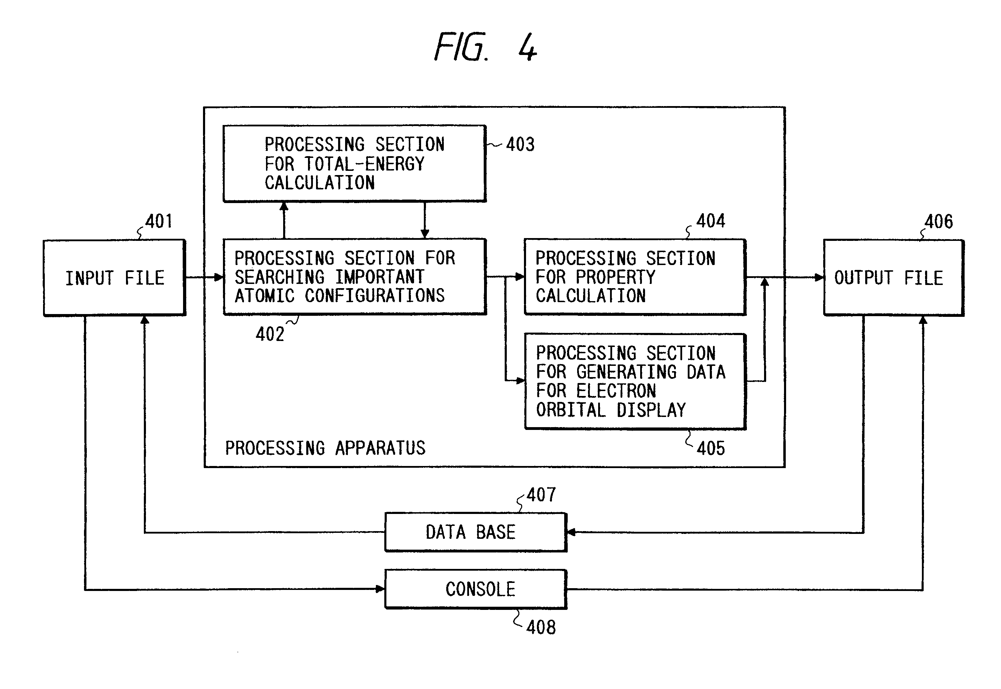

Figure 4 shows the overall configuration of a first embodiment of the material design supporting apparatus according to this invention. In Figure 4, reference numeral 401 signifies an input file to temporarily store experimental data such as compositions and atomic configurations of known materials, entered from data base and console, as well as calculation results produced by this apparatus. Denoted 402 is a processing section to search for important atomic configurations such as stable state and transition state within the material. Designated 403 is a total energy calculation processing section which calculates the electron orbitals and the total energy of the system for the atomic configurations and electronic configurations entered from the processing section 402. Denoted 404 is a processing section for calculating various properties of the system; 405 a processing section for generating data of electron orbital display; 406 an output file that contains the total energy and various properties of the system for important electronic configurations, output from this apparatus; 407 a data base to store, manage and control the data about known materials and results of calculations produced by this apparatus; and 408 a console to display the output data, manage and control the data base, and generate the input data.

Referring to Figure 4, we will explain the flow cf processing as performed by the material design supporting apparatus of the first embodiment. Material data associated with the requested characteristics is selected from among the known materials in the data base 407 and sent to the input file 401. The processing apparatus of this embodiment calculates the electronic orbitals and properties of the material, which are displayed on the console 408. The calculated values and the experimental data of the material are controlled and arranged by the data base 407 and stored therein, and displayed on the console 408. Data of a material with new composition and new structure proposed by a user according to the displayed data are sent through the console 408 to the input file 401. The processing apparatus now calculates important atomic configurations, electron orbitals, total energy and various properties of the newly proposed material and displays them on the console 408. When the calculated result satisfies the requirements, the material design is finished, with the new material registered as a candidate material to be synthesized. If the calculated result fails to satisfy the requirements, the calculation result and the associated data contained in the data base 407 are controlled and managed by the data base 407 before being displayed on the console 408. Then the user proposes another new material according to the displayed data and the similar processing is carried out for the input data of the new material proposed.

One of the features of the material design supporting apparatus and method according to this invention is a method of calculating the total energy of the system by the processing section 403.

The total energy calculation performed in this invention, as shown in Figure 2, involves adding the sum of the orbital energies for specified electronic configurations, i.e. an eigen value of the unperturbed term $E_0$, with an expectation value of perturbation term for the specified electronic configurations, i.e. a first-order correction term $E_1$. This permits a more quantitative calculation than is possible with the conventional extended Hueckel method.

[Second Embodiment: Hydrogen Fluoride]

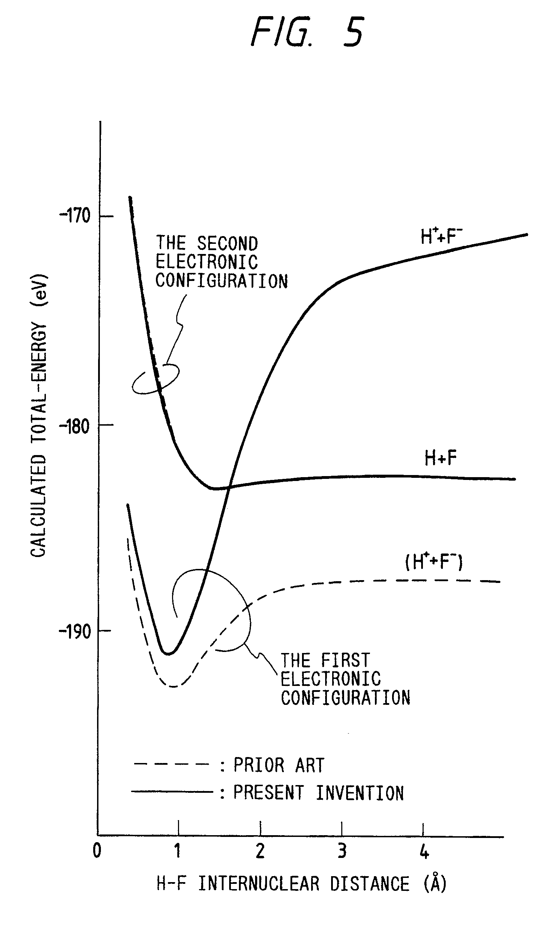

Another embodiment of this invention is described by referring to Figure 5. The overall configuration of the material design supporting apparatus of this embodiment, and the flow of the overall material design processing and the total energy calculation processing are the same as those in the first embodiment.

Figure 5 shows the result of a 0th-order approximation (prior art) and the result of a first-order approximation (this invention) in which the perturbation term of the total Hamiltonian is approximated with the function of excess electron number in each atom. The 0th-order approximation is performed by the LEAF method (known technique, reference 2), using the atomic orbitals of Slater-type. The first-order approximation uses the above-mentioned function $f(R_{\alpha\beta})$ and takes $R_{HF}^{\circ}$ to be 0.9Å. Other parameters are listed in Table 1.

## Table 1

| Ele-ment | Atom orbitals | Zeta | Orbital energy (eV) | Electron repulsion in atom (eV) |
|---|---|---|---|---|
| H | 1s | 1.0000 | -13.60 | 17.00 |
| F | 2s | 2.5639 | -37.86 | 19.53 |
|  | 2p | 2.5500 | -18.65 |  |

Figure 5 shows changes in energy for the first electronic configuration (the ground state at the equilibrium distance) and the second configuration (homo → $\sigma^*$) as the HF internuclear distance changes. As to the second electronic configuration, there is almost no difference between the prior art (0th-order approximation) and the method of this invention (first-order approximation). For the first electronic configuration, however, in the dissociated condition (HF distance > 4Å), there is a significantly large difference between the prior art (dashed line) and the method of this invention (solid line). This means that the conventional method determines the $(H^+ + F^-)$ dissociated state to be more stable than the (H + F) dissociated state, but this is contradictory to the experimental fact. On the contrary, the method of this invention correctly reflects the experimental fact of the dissociated state.

[Third Embodiment: Arsine $AsH_3$]

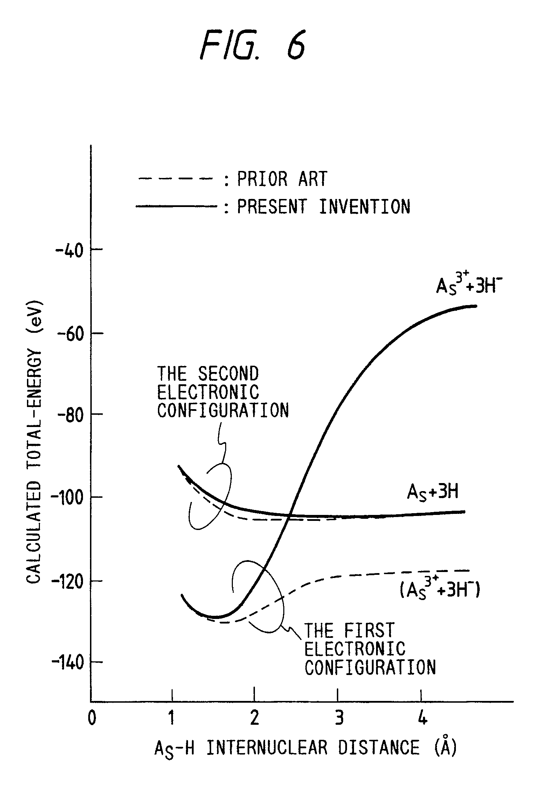

The third embodiment of this invention is explained by referring to Figure 6. The overall configuration of the material design supporting apparatus of this embodiment, and the flow of the overall material design processing and the total energy calculation processing are the same as those in the first embodiment.

Figure 6 shows the result of 0th-order approximation (conventional method) and the result of first-order approximation (method of this invention) in which the perturbation term $\Delta$ of the total Hamiltonian is approximated with the function of excess electron number on each atom. The 0th-order approximation is performed by the LEAF method (known technique, reference 2), using the atomic orbitals of Slater-type. The first-order approximation uses the above-mentioned function $f(R_{\alpha\beta})$ and takes $R_{AsH}$ to be 1.5Å. Other parameters are listed in Table 2.

## Table 2

| Ele-ment | Atom orbitals | Zeta | Orbital energy (eV) | Electron repulsion in atom (eV) |
|---|---|---|---|---|
| H | 1s | 1.0000 | -13.60 | 17.00 |
| As | 4s | 2.2307 | -18.20 | 11.70 |
|  | 4p | 1.8932 | -9.15 |  |

With ∠HAsH fixed at 92.1° and $C_{3v}$ symmetry maintained as is, Figure 6 shows changes in energy for the first electronic configuration (the ground state at the equilibrium distance) and the second electronic configuration (the most stable state at the dissociation as As + 3H) as the AsH inter-nuclear distance changes. As to the second electronic configuration, there is almost no difference between the conventional method (0th-order approximation) and the method of this invention (first-order approximation). For the first electronic configuration, however, in the dissociated condition (AsH distance > 4Å), there is a remarkable difference between the prior art (dashed line) and the method of this invention (solid line). This means that the conventional method determines the ($As^{3+}$ + $3H^-$) dissociated state to be more stable than the (As + 3H) dissociated state, but this is contradictory to the experimental fact. On the contrary, the method of this invention correctly reflects the experimental fact of the dissociated state.

[Fourth Embodiment]

Still anther embodiment of this invention will be explained by referring to Figures 7A and 7B.

An I/O control program 741 in the program memory 704 selects material data associated with the required characteristic from the disc 703 that stores data of known materials, and displays the picked up data on the display 706. At the same time, the program 741 transfers the atomic and electronic configurations of the material from the disc 703 into the work memory 702. As already explained in reference to Figures 1, 7A and 7B, the electronic orbitals of the material and the total energy of the system are calculated and stored in the work memory 702 and disc 703 and also displayed on the display 706. Further, the CPU 701 calculates the properties by a property calculation program 752 in the program memory 704 using the atomic and electronic configurations and electron orbitals contained in the work memory 702. The properties thus obtained are stored in the work memory 702 and the disc 703 and also displayed on the display 706. According to the electron orbitals, total energy and properties of the material, the user proposes a material with new composition and structure from the keyboard 705. The atomic and electronic configurations of the newly proposed material entered from the keyboard 705 are stored in the work memory 702. Along with the total energy calculation processing of this invention, the CPU 701 executes the important atomic configuration searching program 753 and the property calculation program 752, both contained in the program memory 704, by using the atomic and electronic configurations of the newly proposed material stored in the work memory 702, and follows the above-mentioned steps again to determine important atomic and electronic configurations, total energy and properties of the proposed material. The obtained data is then stored in the work memory 702 and also shown on the display 706. When the calculated results are satisfactory, the material design processing is terminated, transferring these data from the work memory 702 to the disc 703 as a candidate material data to be synthesized. If the calculated results are not satisfactory, a processing similar to the one performed when the new material was previously proposed will again be executed by using the associated material data shown on the display 706 and the atomic and electronic configurations and properties of the previously proposed material.

One of the features of the material design supporting method and apparatus according to this invention is the total energy calculation processing performed by programs contained in the program memory 704.

The total energy calculation processing of this invention, as shown in the processing flow of Figure 1, adds the expectation value of the perturbation term for specified electronic configurations, i.e. the first-order correction term $E_1$, to the sum of electron orbital energies for specified electron configurations, i.e. the eigen value of unperturbed term $E_0$. This permits a more quantitative calculation than is possible with the conventional extended Hueckel method.

While in the above explanation the invention uses the first-order approximation of the perturbation term, it is also possible to use other approximations including a second-order perturbation term approximation. The use of a second-order perturbation term approximation, however, will require a longer calculation time.

## Claims

1. A material design supporting method comprising the steps of:
   inputting a first group of parameters defining an atomic configuration of a system made up of a plurality of atoms and a second group of parameters defining carrier configurations in the system;
   determining a one-particle potential common to all carriers according to the first parameter group entered;
   determining carrier orbitals according to the one-particle potential obtained;
   calculating eigen values of unperturbed term making up a Hamiltonian by using the calculated carrier orbitals and the second group of parameters entered, said Hamiltonian representing the total energy of the system; and
   calculating expectation values of perturbation term making up the Hamiltonian by using the carrier orbitals and the second group of parameters.

2. A material design supporting method as claimed in claim 1, wherein the one-particle potential has a form common to all the carriers.

3. A material design supporting method as claimed in claim 1, wherein the second group of parameters includes information on a plurality of carrier configurations.

4. A material design supporting method as claimed in claim 1, wherein the expectation value of the perturbation term is a first-order correction term based on a first-order perturbation approximation.

5. A material design supporting method as claimed in claim 1, wherein the carriers are electrons and the one-particle potential is a Wigner-Seitz-type potential.

6. A material design supporting method as claimed in claim 1, wherein the expectation value of the perturbation term is a function of spin density of atoms.

7. A material design supporting method as claimed in claim 1, wherein the expectation value of the perturbation term is a function of charges of atoms.

8. A material design supporting apparatus comprising:
an input device to input a first group of parameters defining an atomic configuration of a system made up of a plurality of atoms and a second group of parameters defining carrier configurations in the system;
a calculating means for:
determining a one-particle potential common to all carriers according to the first parameter group entered;
determining carrier orbitals according to the one-particle potential obtained;
calculating eigen values of unperturbed term making up a Hamiltonian by using the calculated carrier orbitals and the second group of parameters entered, said Hamiltonian representing the total energy of the system; and
calculating expectation values of perturbation term making up the Hamiltonian by using the carrier orbitals and the second group of parameters; and
a display device to display the expectation values thus obtained.

9. A material design supporting apparatus as claimed in claim 8, wherein the means for calculating the eigen values of the unperturbed term uses the sum of the one-particle Hamiltonians for all the carriers as the unperturbed term in calculating the eigen values.

10. A material design supporting apparatus as claimed in claim 8, wherein the means for calculating the eigen values of the unperturbed term uses a Wigner-Seitz-type potential in calculating eigen values.

11. A material design supporting method comprising the steps of:
approximating a correct total Hamiltonian for a system made up of a plurality of particles with the sum of one-particle Hamiltonians for all particles present in the system, according to parameters that determine the motions of particles in the system;
determining the one-particle Hamiltonian without optimizing it for a particular particle configuration; and
calculating the difference between the correct total Hamiltonian for the system and the sum of one-particle Hamiltonians for all particles in the system by treating the difference as a perturbation term which is common for all particles in the system and by using a perturbation approximation.

12. A material design supporting method as claimed in claim 11, wherein the potential term in the one-particle Hamiltonian for each particle is determined from a Wigner-Seitz-type potential.

13. A material design supporting method as claimed in claim 12, wherein the sum of the one-particle Hamiltonians for all particles is a linkage of the embedded atomic fields or its approximation.

14. A material design supporting method which employs a first-order many-body perturbation theory, in which, when calculating an eigen value for a complete Hamiltonian for a molecule or a cluster of atoms in which all nuclei are fixed in space, i.e. when calculating an eigen value for a total-energy operator

EP 0 431 189 A1

consisting of kinetic energies of all electrons, potentials between nuclei and electrons, inter-electronic potentials, and inter-nuclear potentials; an unperturbed term of the complete Hamiltonian is taken as the sum of one-electron Hamiltonians for all electrons, the one-electron potential being a linkage of one-particle potential functions for free atoms or its approximation, with each one-particle potential term defined on each atomic cell.

15. A material design supporting apparatus which employs a first-order many-body perturbation theory, in which, when calculating an eigen value for a complete Hamiltonian for a molecule or a cluster of atoms in which all nuclei are fixed in space, i.e. when calculating an eigen value for a total-energy operator consisting of kinetic energies of all electrons, potentials between nuclei and electrons, inter-electronic potentials, and inter-nuclear potentials; an unperturbed term of the complete Hamiltonian is taken as the sum of one-electron Hamiltonians for all electrons, the one-electron potential being a linkage of one-particle potential functions for free atoms or its approximation, with each one-particle potential term defined on each atomic cell.

16. A material design supporting method as claimed in claim 14, wherein the expectation value of the perturbation term is the function of spin density of each atom.

17. A material design supporting apparatus as claimed in claim 15, wherein the expectation value of the perturbation term is the function of spin density of each atom.

18. A material design supporting method as claimed in claim 14, wherein the expectation value of the perturbation term is the function of charge of each atom.

19. A material design supporting apparatus as claimed in claim 15, wherein the expectation value of the perturbation term is the function of charge of each atom.

20. A material design supporting method as claimed in claim 1, which uses the one-particle Hamiltonian whose eigen value (or orbital energy) is less than $+0.1$ eV. 21. A material design supporting apparatus as claimed in claim 8, which uses the one-particle Hamiltonian whose eigen value (or orbital energy) is less than $+0.1$ eV.

## FIG. 1

```
                    ┌─────────────┐
                    │    START    │
                    └─────────────┘
                           │
                           ▼
        ┌──────────────────────────────────────────┐  ┌101
        │      READING AN ATOMIC CONFIGURATION      │
        └──────────────────────────────────────────┘
                           │
                           ▼
        ┌──────────────────────────────────────────┐  ┌102
        │      GENERATION OF MATRIX ELEMENTS OF LEAF │
        │      ONE-ELECTRON HAMILTONIAN             │
        └──────────────────────────────────────────┘
                           │
                           ▼
        ┌──────────────────────────────────────────┐  ┌103
        │      CALCULATION AND OUTPUTTING THE ELECTRON │
        │      ORBITAL FUNCTIONS                     │
        └──────────────────────────────────────────┘
                           │
                           ▼
        ┌──────────────────────────────────────────┐  ┌104
        │      READING ELECTRONIC CONFIGURATION      │
        └──────────────────────────────────────────┘
                           │
                           ▼
        ┌──────────────────────────────────────────┐  ┌105
        │      CALCULATION OF THE RESPECTIVE EIGEN   │
        │      VALUES OF THE UNPERTURBED TERM (E_0) FOR │
        │      ELECTRONIC CONFIGURATIONS            │
        └──────────────────────────────────────────┘
                           │
                           ▼
        ┌──────────────────────────────────────────┐  ┌106
        │      CALCULATION OF THE RESPECTIVE EXPECTATION │
        │      VALUE OF THE PERTURBATION TERM (E_1) FOR │
        │      ELECTRONIC CONFIGURATIONS            │
        └──────────────────────────────────────────┘
                           │
                           ▼
        ┌──────────────────────────────────────────┐  ┌107
        │   TOTAL-ENERGY CALCULATION BY ADDING E_1 TO E_0 │
        └──────────────────────────────────────────┘
                           │
                           ▼
        ┌──────────────────────────────────────────┐  ┌108
        │      OUTPUTTING THE RESPECTIVE TOTAL ENERGIES │
        │      FOR ELECTRONIC CONFIGURATIONS        │
        └──────────────────────────────────────────┘
                           │
                           ▼
                    ┌─────────────┐
                    │     END     │
                    └─────────────┘
```

## FIG. 2

EP 0 431 189 A1

FIG. 3

FIG. 4

PROCESSING SECTION FOR TOTAL-ENERGY CALCULATION ~403

INPUT FILE ~401

PROCESSING SECTION FOR SEARCHING IMPORTANT ATOMIC CONFIGURATIONS 402~

PROCESSING SECTION FOR PROPERTY CALCULATION ~404

PROCESSING SECTION FOR GENERATING DATA FOR ELECTRON ORBITAL DISPLAY ~405

OUTPUT FILE ~406

PROCESSING APPARATUS

DATA BASE ~407

CONSOLE ~408

EP 0 431 189 A1

# FIG. 5

# FIG. 6

## FIG. 7A

CPU 701

WORK MEMORY 702

DISC MEMORY 703

PROGRAM MEMORY 704

INPUT DEVICE 705

OUTPUT DEVICE 706

EP 0 431 189 A1

# FIG. 7B

704

| | |
|---|---|
| I/O CONTROL PROGRAM | 741 |
| WORK MEMORY MANAGEMENT PROGRAM | 742 |
| OVERLAP INTEGRAL CALCULATION PROGRAM | 743 |
| INNER-CELL INTEGRAL CALCULATION PROGRAM | 744 |
| ATOMIC ORBITAL PARAMETER | 745 |
| DIAGONAL MATRIX OF ATOMIC ORBITAL ENERGY GENERATION PROGRAM | 746 |
| MATRIX MULTIPLICATION PROGRAM | 747 |
| MATRIX TRANSFORMATION PROGRAM | 748 |
| MATRIX DIAGONALIZATION PROGRAM | 749 |
| EIGEN VALUE CALCULATION PROGRAM | 750 |
| EXPECTATION VALUE CALCULATION PROGRAM | 751 |
| PROPERTY CALCULATION PROGRAM | 752 |
| IMPORTANT ATOMIC CONFIGURATION SEARCHING PROGRAM | 753 |

# INTERNATIONAL SEARCH REPORT

International Application No PCT/JP90/00822

## I. CLASSIFICATION OF SUBJECT MATTER (if several classification symbols apply, indicate all) 6

According to International Patent Classification (IPC) or to both National Classification and IPC

Int. Cl$^5$    G01N33/00

## II. FIELDS SEARCHED

Minimum Documentation Searched 7

| Classification System | Classification Symbols |
|---|---|
| IPC | G01N33/00, G06F15/60-15/62 |

Documentation Searched other than Minimum Documentation
to the Extent that such Documents are Included in the Fields Searched 8

| | |
|---|---|
| Jitsuyo Shinan Koho | 1926 - 1990 |
| Kokai Jitsuyo Shinan Koho | 1971 - 1990 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 1-112373 (Fujitsu Ltd.; Fuji Facom Seigyo K.K.), 1 May 1989 (01. 05. 89), Lines 9 to 16, upper right column, page 2 (Family: none) | 1 - 21 |
| A | JP, A, 1-161578 (Toshiba Corp.), 26 June 1989 (26. 06. 89), Line 20, lower right column, page 1 to line 20, upper left column, page 2 (Family: none) | 1 - 21 |
| A | Theoretica Chimica Acta (1986) 70, p.239-252 | 1 - 21 |

* Special categories of cited documents: 10
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

## IV. CERTIFICATION

| Date of the Actual Completion of the International Search | Date of Mailing of this International Search Report |
|---|---|
| August 28, 1990 (28. 08. 90) | September 10, 1990 (10. 09. 90) |

| International Searching Authority | Signature of Authorized Officer |
|---|---|
| Japanese Patent Office | |

Form PCT/ISA/210 (second sheet) (January 1985)